(19) Europäisches Patentamt
European Patent Office
Office européen des brevets

(11) EP 4 023 211 A1

(12) EUROPEAN PATENT APPLICATION

(43) Date of publication:
06.07.2022   Bulletin 2022/27

(21) Application number: 21218381.8

(22) Date of filing: 30.12.2021

(51) International Patent Classification (IPC):
A61K 9/127 (2006.01)        A61K 9/51 (2006.01)

(52) Cooperative Patent Classification (CPC):
A61K 9/1271; A61K 9/5115; A61K 9/5146;
A61K 31/00

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority:   31.12.2020   US 202063132501 P

(71) Applicant: Taipei Medical University
Taipei City 110 (TW)

(72) Inventors:
• WU, Si-Han
  110 TAIPEI CITY (TW)
• CHUANG, Kuo-Hsiang
  110 TAIPEI CITY (TW)
• CHEN, Yi-Ping
  110 TAIPEI CITY (TW)

(74) Representative: Lavoix
Bayerstraße 83
80335 München (DE)

(54) **HIGH-DENSITY AND SHORT-CHAIN PEG MODIFIED NANO-SIZED CARRIERS AND THEIR USES**

(57)    The present disclosure relates to PEGylated surface, in particular nano-sized carriers with such surface, in medical applications, such as a drug delivery system. Particularly, the PEGylated nano-sized carriers may solve the immunological issues caused by or related to polyethylene glycol-containing substances.

EP 4 023 211 A1

**Description**

**FILED OF THE INVENTION**

The present disclosure relates to nano-sized carriers with PEGylated surface and the use thereof in escaping from recognition of anti-PEG antibodies.

**BACKGROUND OF THE INVENTION**

Nano-sized carriers for delivering bioactive ingredients such as drugs, labeling agents, etc., have been widely used. These carriers include, but are not limited to, liposomes, nanoparticles, micelles, etc. These carriers are considered efficient means to deliver active ingredients for ameliorating or treating symptoms and diseases.

One of the strategies for targeted drug distribution is to encapsulate the drug in nanoparticles, as mentioned earlier. To enhance bioavailability of the nanoparticles, it is common to use poly(ethylene glycol) (PEG) to modify the surface of the nanoparticles. These nanocarriers would carry the drug "payload" within and deliver it to the intended target site.

However, recent research reveals that anti-PEG antibodies may be present in the human body due to the frequent use of PEG modifications, not only in clinical applications but also in common life such as cosmetics, etc. The anti-PEG antibodies may bind to PEGylated substances or agents such as therapeutic agents, vaccines, carriers, etc.,that are administered to a subject and thus reduce the effect of these PEGylated substances and induce (pseudo-)anaphylaxis. Therefore, there is a need to develop a solution to the abovementioned problems.

**SUMMARY OF THE INVENTION**

The inventors surprisingly found that nano-sized carriers having specific surface modification with short-chain poly(ethylene glycol) (PEG) that have good dispersity without aggregation (DLS size <200 nm) in aqueous solution, PBS or physiological condition and would not be identified by anti-PEG antibodies so that such carriers can have an increased effect in pharmaceutical applications.

In one aspect, the present disclosure provides a method for preventing induction of producing an anti-PEG antibody or identification by an anti-PEG antibody in a subject, comprising loading a bioactive agent to a nano-sized carrier having surface modification with short-chain PEG and administering the PEGylated nano-sized carrier with the loaded bioactive agent to a subject, wherein the short-chain PEG has no greater than 45 repeating units of oxyethylene ($-O-CH_2-CH_2-$), e.g., the short-chain PEG having a structure of $(-O-CH_2-CH_2-)_n$ wherein n is no greater than 45. Alternatively, the present disclosure provides a method of using a nano-sized carrier having surface modification with short-chain PEG to prevent induction of producing an anti-PEG antibody or identification by an anti-PEG antibody, wherein the nano-sized carrier is loaded with a bioactive agent and the short-chain PEG has less than 45 repeating units of oxyethylene ($-O-CH_2-CH_2-$), e.g., the short-chain PEG having a structure of $(-O-CH_2-CH_2-)_n$ wherein n is no greater than 45. Alternatively, the present disclosure provides a nano-sized carrier for preventing induction of producing an anti-PEG antibody or preventing identification by an anti-PEG antibody, wherein the nano-sized carrier is loaded with a bioactive agent and the nano-sized carrier has surface modification with short-chain PEG short-chain having no greater than 45 repeating units of oxyethylene ($-O-CH_2-CH_2-$), e.g., the short-chain PEG having a structure of $(-O-CH_2-CH_2-)_n$ wherein n is no greater than 45. Alternatively, the present disclosure provides a use of a nano-sized carrier in the manufacture of a preparation for preventing induction of producing an anti-PEG antibody or identification by an anti-PEG antibody, wherein the nano-sized carrier is loaded with a bioactive agent and the nano-sized carrier has surface modification with short-chain PEG short-chain having no greater than 45 repeating units of oxyethylene $(-O-CH_2-CH_2-)_n$, e.g., the short-chain PEG having a structure of $(-O-CH_2-CH_2-)_n$ wherein n is no greater than 45.

In one embodiment, the short-chain PEG has about 1 to about 45 repeating units of oxyethylene. In some embodiments, the short-chain PEG has, about 5 to about 45, about 5 to about 40, about 5 to about 35, about 5 to about 30, about 5 to about 25, about 5 to about 20, about 5 to about 15, about 5 to about 10, about, about 6 to about 45, about 6 to about 40, about 6 to about 35, about 6 to about 30, about 6 to about 25, about 6 to about 20, about 6 to about 15, about 6 to about 10, , about 7 to about 45, about 7 to about 40, about 7 to about 35, about 7 to about 30, about 7 to about 25, about 7 to about 20, about 7 to about 15, about 7 to about 10, about 8 to about 45, about 8 to about 40, about 8 to about 35, about 8 to about 30, about 8 to about 25, about 10 to about 45, about 10 to about 40, about 10 to about 35, about 10 to about 30, about 10 to about 25, about 10 to about 20, about 15 to about 45, about 15 to about 40, about 15 to about 35, about 15 to about 30, about 15 to about 25, about 20 to about 45, about 20 to about 40, about 20 to about 35, about 20 to about 30, about 25 to about 45, about 25 to about 40, about 25 to about 35, about 30 to about 45 or about 30 to about 40 repeating units of oxyethylene.

In one embodiment, the short-chain PEG as described herein has a molecular weight of less than 2,000 Da, 1,500 Da, 1,200 Da, 1,000 Da or 750 Da. In some further embodiments, the short-chain PEG has a molecular weight ranging from about 300 Da to about 2,000 Da, about 300 Da to about 1,800 Da, about 300 Da to about 1,500 Da, about 300 Da to about 1,000 Da, about 300 Da to about 750 Da, about 300 Da to about 500 Da, about 350 Da to about 2,000 Da, about 350 Da to about 1,800 Da, about 350 Da to about 1,500 Da, about 350 Da to about 1,000 Da, about 350 Da to about 750 Da, about 350 Da to about 500 Da, about 400 Da to about 2,000 Da, about 400 Da to about 1,800 Da, about 400 Da to about 1,500 Da, about 400 Da to about 1,000 Da, about 400 Da to about 750 Da, about 450 Da to about 2,000 Da, about 450 Da to about 1,800 Da, about 450 Da to about 1,500 Da, about 450 Da to about 1,000 Da, about 450 Da to about 750 Da, about 500 Da to about 2,000 Da, about 500 Da to about 1,800 Da, about 500 Da to about 1,500 Da, about 500 Da to about 1,000 Da or about 500 Da to about 750 Da.

In one embodiment, the density of the short-chain PEG as described herein on the surface of the nano-sized carrier is no less than 0.5, 1, 2, 3, 4, 5 or 6 short-chain PEG molecule/nm$^2$; hereinafter the unit can be abbreviated as "PEG/nm$^2$" or "/nm$^2$" for conciseness. In some embodiments, the density of the short-chain PEG as described herein on the surface of the nano-sized carrier ranges from about 0.5/nm$^2$ to about 7/nm$^2$, about 0.5/nm$^2$ to about 6/nm$^2$, about 0.5/nm$^2$ to about 5/nm$^2$, about 0.5/nm$^2$ to about 4.5/nm$^2$, about 0.5/nm$^2$ to about 4/nm$^2$, about 0.5/nm$^2$ to about 3.5/nm$^2$, about 0.5/nm$^2$ to about 3/nm$^2$, about 0.5/nm$^2$ to about 2.5/nm$^2$, about 0.5/nm$^2$ to about 2/nm$^2$, about 0.8/nm$^2$ to about 5/nm$^2$, about 0.8/nm$^2$ to about 4.5/nm$^2$, about 0.8/nm$^2$ to about 4/nm$^2$, about 0.8/nm$^2$ to about 3.5/nm$^2$, about 0.8/nm$^2$ to about 3/nm$^2$, about 0.8/nm$^2$ to about 2.5/nm$^2$, about 0.8/nm$^2$ to about 2/nm$^2$, about 1/nm$^2$ to about 7/nm$^2$, about 1/nm$^2$ to about 6/nm$^2$, about 1/nm$^2$ to about 5/nm$^2$, about 1/nm$^2$ to about 4.5/nm$^2$, about 1/nm$^2$ to about 4/nm$^2$, about 1/nm$^2$ to about 3.5/nm$^2$, about 1/nm$^2$ to about 3/nm$^2$, about 1/nm$^2$ to about 2.5/nm$^2$, about 1/nm$^2$ to about 2/nm$^2$, about 1.5/nm$^2$ to about 7/nm$^2$, about 1.5/nm$^2$ to about 6/nm$^2$, about 1.5/nm$^2$ to about 5/nm$^2$, about 1.5/nm$^2$ to about 4.5/nm$^2$, about 1.5/nm$^2$ to about 4/nm$^2$, about 1.5/nm$^2$ to about 3.5/nm$^2$, about 1.5/nm$^2$ to about 3/nm$^2$, about 1.5/nm$^2$ to about 2.5/nm$^2$, about 2/nm$^2$ to about 7/nm$^2$, about 2/nm$^2$ to about 6/nm$^2$, about 2/nm$^2$ to about 5/nm$^2$, about 2/nm$^2$ to about 4.5/nm$^2$, about 2/nm$^2$ to about 4/nm$^2$, about 2/nm$^2$ to about 3.5/nm$^2$, about 2/nm$^2$ to about 3/nm$^2$, about 2.5/nm$^2$ to about 7/nm$^2$, about 2.5/nm$^2$ to about 6/nm$^2$, about 2.5/nm$^2$ to about 5/nm$^2$, about 2.5/nm$^2$ to about 4.5/nm$^2$, about 2.5/nm$^2$ to about 4/nm$^2$, about 2.5/nm$^2$ to about 3.5/nm$^2$, about 3/nm$^2$ to about 7/nm$^2$, about 3/nm$^2$ to about 6/nm$^2$, about 3/nm$^2$ to about 5/nm$^2$, about 4/nm$^2$ to about 7/nm$^2$, about 4/nm$^2$ to about 6 /nm$^2$, or about 4/nm$^2$ to about 5 /nm$^2$.

In one embodiment, the PEGylated nano-sized carrier having the short-chain PEG as described herein has an apparent size ranging from about 10 nm to about 200 nm, about 10 nm to about 180 nm, about 10 nm to about 150 nm, about 10 nm to about 120 nm, about 10 nm to about 100 nm, about 10 nm to about 80 nm, about 10 nm to about 60 nm, about 10 nm to about 50 nm, about 20 nm to about 200 nm, about 20 nm to about 180 nm, about 20 nm to about 150 nm, about 20 nm to about 120 nm, about 20 nm to about 100 nm, about 20 nm to about 80 nm, about 20 nm to about 60 nm, about 20 nm to about 50 nm, about 50 nm to about 200 nm, about 50 nm to about 180 nm, about 50 nm to about 150 nm, about 50 nm to about 120 nm or about 50 nm to about 100 nm.

In one embodiment, the prevention of inducing production of an anti-PEG antibody or identification by an anti-PEG antibody can reduce or eliminate binding of a PEGylated nano-sized carrier to an anti-PEG antibody and/or prevent reduction of effect or efficacy of a PEGylated bioactive agent or carrier or reduce adverse events caused by the anti-PEG antibody.

In one embodiment, the PEGylated nano-sized carriers described herein can be organic, inorganic, polymeric and metallic nanostructures, including, but not limited to, liposomes, lipid nanoparticles, metal nanoparticles, micelles, dendrimers, polymers and silica nanoparticles. In a further embodiment, the PEGylated nano-sized carriers are silica nanoparticles. In another embodiment, the nanoparticles described herein are solid or hollow. In another embodiment, the hollow nanoparticles have pores with less than 15 nm pore diameter. In another embodiment, the nano-sized carrier is a mesoporous inorganic nanoparticle (such as silica nanoparticles), liposome or a metal (oxide) nanoparticle.

In one embodiment, the mesoporous inorganic nanoparticle is a mesoporous silica nanoparticle. In one embodiment, the metal of the metal (oxide) nanoparticle is selected from the group consisting of gold, silver, copper, zinc, iron, aluminum, manganese, nickel, titanium dioxide, cerium, platinum, calcium, bismuth, chromium.

In one embodiment, the nano-sized carrier described herein has a DLS particle size ranging from 10 nm to 200 nm, 10 nm to 150 nm, 10 nm to 100 nm, 10 nm to 75nm, 10 nm to 60 nm, 10 nm to 50 nm, 25 nm to 200 nm, 25 nm to 150 nm, 25 nm to 100 nm, 25 nm to 75 nm, 25 nm to 60 nm, 25 nm to 50 nm in aqueous solutions, PBS or physiological condition.

In one embodiment, the bioactive agent described herein can be non-PEGylated or PEGylated. In one embodiment, the bioactive agent comprises, but is not limited to, a small molecule drug (such as an antibiotic and an anticancer drug), a large molecule drug (such as an antibody, a DNA drug, an RNA drug, a protein drug or a peptide drug), a vaccine, an enzyme and an immunogen.

In one aspect, the present disclosure also provides a nano-sized carrier having surface modification with short-chain PEG, wherein the short-chain PEG has no more than 17 repeating units of oxyethylene (-O-CH$_2$-CH$_2$-)$_n$ and has a density on the surface of the nano-sized carrier of no less than 0.5 /nm$^2$. In some embodiment, the short-chain PEG has a density on the surface of the nano-sized carrier from 0.5 to 7/nm$^2$ or 0.5 to 5/nm$^2$.

In one embodiment, the short-chain PEG described herein has about 5 to about 17, about 5 to about 15, about 5 to about 12, about 5 to about 10, about 6 to about 15, about 6 to about 12 or about 6 to about 10 repeating units of oxyethylene.

In one embodiment, the short-chain PEG described herein has a molecular weight of no more than 1,000 Da. In some further embodiments, the short-chain PEG described herein has a molecular weight ranging from about 300 Da to about 1,000 Da, about 300 Da to about 750 Da, about 300 Da to about 500 Da, about 350 Da to about 1,000 Da, about 350 Da to about 750 Da, about 350 Da to about 500 Da, about 400 Da to about 1,000 Da, about 400 Da to about 750 Da, about 450 Da to about 1,000 Da, about 450 Da to about 750 Da, about 500 Da to about 1,000 Da or about 500 Da to about 750 Da.

In some embodiments, the density of the short-chain PEG as described herein on the surface of the nano-sized carrier ranges from about $0.5/nm^2$ to about $5/nm^2$, about $0.5/nm^2$ to about $4/nm^2$, about $0.5/nm^2$ to about $3/nm^2$, about $0.5/nm^2$ to about $2.5/nm^2$, about $0.5/nm^2$ to about $2/nm^2$, about $0.8/nm^2$ to about $5/nm^2$, about $0.8/nm^2$ to about $4/nm^2$, about $0.8/nm^2$ to about $3/nm^2$, about $0.8/nm^2$ to about $2.5/nm^2$, about $0.8/nm^2$ to about $2/nm^2$, about $1/nm^2$ to about $5/nm^2$, about $1/nm^2$ to about $4/nm^2$, about $1/nm^2$ to about $3/nm^2$, about $1/nm^2$ to about $2.5/nm^2$, about $1/nm^2$ to about $2/nm^2$, about $1.5/nm^2$ to about $5/nm^2$, about $1.5/nm^2$ to about $4/nm^2$, about $1.5/nm^2$ to about $3/nm^2$, about $1.5/nm^2$ to about $2.5/nm^2$, about $2/nm^2$ to about $5/nm^2$, about $2/nm^2$ to about $4/nm^2$, about $2/nm^2$ to about $3.5/nm^2$ or about $2/nm^2$ to about $3/nm^2$.

In some embodiments, the PEGylated nano-sized carrier having the short-chain PEG as described herein has an apparent size as described herein.

In one embodiment, the PEGylated nano-sized carriers described herein can be organic, inorganic, polymeric and metallic nanostructures, including, but not limited to, liposomes, lipid nanoparticles, metal nanoparticles, micelles, dendrimers, polymers and silica nanoparticles. In a further embodiment, the PEGylated nano-sized carriers are silica nanoparticles. In another embodiment, the nanoparticles described herein are solid or hollow. In another embodiment, the hollow nanoparticles have pores on surface or shell with a pore diameter of less than 15 nm. In another embodiment, the nano-sized carrier is a mesoporous inorganic nanoparticle (such as silica nanoparticles), liposome or a metal (oxide) nanoparticle.

In one embodiment, the mesoporous inorganic nanoparticle is a mesoporous silica nanoparticle. In one embodiment, the metal of the metal (oxide) nanoparticle is selected from the group consisting of gold, silver, copper, zinc, iron, aluminum, manganese, nickel, titanium dioxide, cerium, platinum, calcium, bismuth, chromium.

In one embodiment, the PEGylated nano-sized carrier is prepared by a method comprising the steps of: adding about 0.02 w/v to about 1.0 w/v (preferably about 0.1 w/v to about 0.6 w/v) of PEG solution to nano-sized carriers to form a desired density, aging the resulting mixture, placing it in an oven for more than 24 hours of hydrothermal treatment at about 80°C to about 95°C to form nano-carrier carrier with a desired PEG density. In some embodiment, the concentration of the PEG solution is about 0.02 w/v to about 1.0 w/v, about 0.02 w/v to about 0.8 w/v, about 0.02 w/v to about 0.6 w/v, about 0.02 w/v to about 0.4 w/v, about 0.02 w/v to about 0.2 w/v, about 0.02 w/v to about 0.1 w/v, about 0.02 w/v to about 0.08 w/v, about 0.02 w/v to about 0.06 w/v, about 0.04 w/v to about 1.0 w/v, about 0.04 w/v to about 0.8 w/v, about 0.04 w/v to about 0.6 w/v, about 0.04 w/v to about 0.4 w/v, about 0.04 w/v to about 0.2 w/v, about 0.04 w/v to about 0.1 w/v, about 0.04 w/v to about 0.08 w/v, about 0.04 w/v to about 0.06 w/v, about 0.06 w/v to about 1.0 w/v, about 0.06 w/v to about 0.8 w/v, about 0.06 w/v to about 0.6 w/v, about 0.06 w/v to about 0.4 w/v, about 0.06 w/v to about 0.2 w/v, about 0.06 w/v to about 0.1 w/v, about 0.06 w/v to about 0.08 w/v, about 0.08 w/v to about 1.0 w/v, about 0.08 w/v to about 0.8 w/v, about 0.08 w/v to about 0.6 w/v, about 0.08 w/v to about 0.4 w/v, about 0.08 w/v to about 0.2 w/v, about 0.08 w/v to about 0.1 w/v, about 0.1 w/v to about 1.0 w/v, about 0.1 w/v to about 0.8 w/v, about 0.1 w/v to about 0.6 w/v, about 0.1 w/v to about 0.4 w/v, about 0.1 w/v to about 0.2 w/v, about 0.2 w/v to about 1.0 w/v, about 0.2 w/v to about 0.8 w/v, about 0.2 w/v to about 0.6 w/v, about 0.2 w/v to about 0.4 w/v, about 0.4 w/v to about 1.0 w/v, about 0.4 w/v to about 0.8 w/v, about 0.4 w/v to about 0.6 w/v, about 0.6 w/v to about 1.0 w/v, about 0.6 w/v to about 0.8 w/v or about 0.8 w/v to about 1.0 w/v.

In one embodiment, the PEGylated nano-sized carrier described herein is an inorganic non-metal or metallic nanoparticle. In one embodiment, the PEGylated nano-sized carrier described herein is an organic or inorganic non-metal nanoparticle. In one embodiment, the PEGlyated nano-sized carrier described herein is an inorganic non-metal nanoparticle. In one embodiment, the PEGylated nano-sized carrier described herein is an inorganic non-metal or metallic nanoparticle, and the molecular weight of short-chain PEG is less than 2,000 Da. In one embodiment, the PEGylated nano-sized carrier described herein is an organic or inorganic non-metal nanoparticle, and the molecular weight of short-chain PEG is less than 2,000 Da. In one embodiment, the PEGylated nano-sized carrier described herein is an inorganic non-metal nanoparticle and the molecular weight of short-chain PEG is less than 2,000 Da. In one embodiment, the PEGylated nano-sized carrier described herein is an inorganic non-metal or metallic nanoparticle, the molecular weight of short-chain PEG is less than 2,000 Da, and the density of the short-chain PEG on the surface of the nano-sized carrier is no less than $0.5/nm^2$. In one embodiment, the PEGylated nano-sized carrier described herein is an organic or inorganic non-metal nanoparticle, the molecular weight of short-chain PEG is less than 1,000 Da, and the density of the short-chain PEG on the surface of the nano-sized carrier is no less than $0.5/nm^2$. In one embodiment, the PEGylated nano-sized carrier described herein is an inorganic non-metal nanoparticle, the molecular weight of short-chain PEG described herein is less than 1,000 Da, and the density of the short-chain PEG on the surface of the nano-sized carrier is no less than $0.5/nm^2$. In one embodiment, the PEGylated nano-sized carrier described herein is a mesoporous silica or organic liposomal nanoparticle, the molecular weight of short-chain PEG is less than 1,000 Da, and the density of the short-chain PEG on the surface of the nano-sized carrier is no less than $0.5/nm^2$. In one embodiment, the PEGylated nano-sized carrier described herein is a mesoporous silica nanoparticle, the molecular weight of short-chain PEG is less than 1,000 Da, and the density of the short-chain PEG on the surface of the nano-sized carrier is no less than $0.5/nm^2$. In one embodiment, the PEGylated nano-sized carrier described herein is an organic liposomal nanoparticle, the molecular weight of short-chain PEG is less than 1,000 Da, and the density of the short-chain PEG on the surface of the nano-sized carrier is no less than $0.5/nm^2$. The further embodiments of the molecular weight of the short-chain PEG or density of the short-chain PEG on the surface of the nano-sized carrier or the material of the nano-sized carrier mentioned in this paragraph can be those mentioned in previous paragraphs described herebefore.

In yet another aspect, the present disclosure relates to a drug delivery system, comprising the nano-sized carriers and a bioactive agent as described herein

## BRIEF DESCRIPTIONS OF THE DRAWINGS

Figs. 1A to 1C show DLS measurements of Doxil, liposome-PEG2000 and liposome-PEG550 in water and PBS.
Figs. 2A to 2D show characteristics of the short-chain PEG functionalized MSNs disclosed herein.
Fig. 3 shows TEM images of AuNP-PEG500 and AuNP-PEG2000 with different PEG densities. HML is defined as high-, medium- or low-density of PEG modification
Figs. 4A and 4B show the specific binding affinity between PEG-coated nanoparticles and anti-PEG antibodies by ELISA.
Figs. 5A to 5C show the *in vivo* results of using PEGylated liposomal drug and the inventive short-chain PEG functionalized MSNs.
Figs 6A to 6D show the adverse effects of treatment on mice with PEGylated drugs.

## DETAILED DESCRIPTION OF THE INVENTION

In order to facilitate the understanding of the disclosure herein, terms as used herein are hereby defined below.

In the context of the specification and the claims, the singular forms "a," "an" and "the" include plural referents, unless specifically indicated otherwise. Unless otherwise stated, any example or exemplary language (e.g., "such as") provided herein is merely used for better illustration of the present disclosure, instead of limiting the scope of the present disclosure.

It is to be understood that any numerical range recited in this specification is intended to include all sub-ranges encompassed therein. For example, a range from "50 to 70°C" includes all sub-ranges and specific values between the stated minimum value of 50°C and the stated maximum value of 70°C, inclusive, e.g., from 58°C to 67°C, and from 53°C to 62°C, 60°C or 68°C. Since the numerical ranges disclosed are continuous, they contain each numerical value between the minimum and maximum values. Unless otherwise specified, the various numerical ranges indicated in this specification are approximate.

In the present disclosure, the term "about" refers to an acceptable deviation of a given value measured by a person of ordinary skill in the art, depending, in part, on how to measure or determine the value.

In the present disclosure, unless particularly specified, the prefix "nano" as used herein means a size of about 300 nm or less. Unless particularly specified, the prefix "meso" as used herein, unlike the definition suggested by IUPAC, means a size of about 10 nm or less.

In the present disclosure, the term "silane" as used herein refers to derivatives of $SiH_4$. Normally, at least one of the four hydrogens is replaced with substituents such as alkyl, alkoxyl, amino, etc., as described below. The term "alkoxysilane" as used herein refers to a silane having at least one alkoxyl substituent directly bonded to the silicon atom. The term "organo-alkoxysilane" as used herein refers to a silane having at least one alkoxyl substituent and at least one hydrocarbyl substituent directly bonded to the silicon atom. The term "silicate source" as used herein refers to substances which can be deemed as a salt form or an ester form of orthosilicic acid, for example, sodium orthosilicate, sodium metasilicate, tetraethyl orthosilicate (tetraethoxy silane, TEOS), tetramethyl orthosilicate, tetrapropyl orthosilicate. Optionally, the hydrocarbyl substituent can be further substituted or interrupted with a heteroatom.

In the present disclosure, the term "bioactive agent" as used herein refers to a substance having an activity in an organism.

Polyethylene glycol (PEG), a biocompatible polymer routinely used in protein and nanoparticle therapeutics, is a polymer routinely used to modify biologics and nanoparticles to prolong blood circulation and reduce immunogenicity of the underlying therapeutic. However, several PEGylated therapeutics induce the development of anti-PEG antibodies (APA), leading to reduced efficacy and increased adverse events. It is known that circulating antibodies (Ab) that specifically bind PEG have been associated with reduced efficacy of and/or adverse reactions to therapeutics modified with or containing PEG. A recent study showed that anti-PEG antibodies were detected in 44.3% of healthy donors with a high prevalence of both anti-PEG IgM (27.1%) and anti-PEG IgG (25.7%) (Analytical Chemistry 2016, 88 (21), 10661-10666.) Anti-PEG IgM and IgG antibodies were significantly more common in females than males. The prevalence of anti-PEG antibodies was higher in younger populations than older ones. After COVID 19 vaccination, this frequency will certainly increase due to the extensive use of PEGylated LNP (lipid-nano-particle) in mRNA vaccine. The loss of efficacy of PEGylated nanodrug due to anti-PEG antibodies should be aware of and closely monitored.

Many therapeutic agents and vaccines are modified with PEG molecules. For example, Oncaspar[®] (a PEG-asparaginase) can be used for treating acute lymphoblastic leukemia (ALL); Doxil[®] (a liposomal doxorubicin) can be used for treating Kaposi's sarcoma and cancer tumors; Onivyde[®] (a liposomal irinotecan) can be used for treating metastatic pancreatic cancer. Vaccines, such as COVID-19 vaccines, e.g., Pfizer-BioNTech (BNT) vaccine (using 2-(PEG2000)-N,N-ditetradecylacetamide) and Moderna vaccine (using PEG2000-DMG), also involve PEG molecules. It is generally known that PEGylation of nanoparticles increases the circulation time by preventing them from being detected by the body's immune system (a trait known as "stealth"). However, increasing evidence suggests that PEGylation can cause an immune response from the body; and there exist complex interactions between PEGylated nanoparticles and the immune system, as discussed above. These interactions may instead have an undesired effect of reducing the efficacy of the treatment. The injection of PEGylated nanoparticles into a subject can elicit an immune response that induces the production of what is known as "anti-PEG antibodies." These antibodies persist in the bloodstream and bind to subsequently injected nanoparticles, resulting in rapid clearance from the body in a process called the Accelerated Blood Clearance phenomenon (ABC phenomenon). Another risk given said immune response is inducing (pseudo-) anaphylaxis, which may even cause death. Interestingly enough, PEG alone does not produce anti-PEG antibodies, which implies that PEG alone may only act as a hapten, not an antigen. Such therapeutic agents and vaccines would induce the generation of anti-PEG antibodies in a subject. These antibodies reduce efficacy and/or induce adverse reactions to therapeutics modified with or containing PEG. It would be important to prevent or solve the problems caused by the anti-PEG antibodies. The current study (Communications Chemistry 2020, 3 (1), 124.) shows that APAs can bind highly flexible repeated ($-O-CH_2-CH_2-$) structure that lacks fixed conformations. By contrast, a soluble PEG, i.e., PEG is not immobilized on one end, of less than 4000 Da is almost undetectable by sandwich ELISA. (ACS Nano 2021, 15 (9), 14022-14048.) Therefore, given the highly flexible structure of PEG or completely immobilized PEG attached to nanoparticles, how APA specifically bind PEG remains poorly understood.

Surprisingly, the present disclosure uses a nano-sized carrier having surface modification with short-chain PEG to prevent induction of producing an anti-PEG antibody or identification by an anti-PEG antibody, wherein the nano-sized carrier is loaded with a non-PEGylated or PEGylated bioactive agent and the short-chain PEG has less than 45 repeating units of oxyethylene ($-O-CH_2-CH_2-)_n$. Such molecular weight-based design of PEGylation of a nano-sized carrier can achieve better immune tolerance and improve the safe and timely use of PEGylated nanomedicine and nanovaccine.

The nano-sized carriers described herein can be organic, inorganic, polymeric and metallic nanostructures, including liposomes, lipid nanoparticles, micelles, dendrimers, polymers and silica nanoparticles.

Liposomes are spherical vesicles having at least one lipid bilayer. The lipid layer can be composed of, e.g., phospholipids, especially phosphatidylcholine, but may also include other lipids, such as egg phosphatidylethanolamine. PEG modification on liposomes has been reported and commercialized, but the abovementioned problems and risks may still be challenging. PEGylated liposomal doxorubicin (Doxil[®]), irinotecan (Onivyde[®]) and siRNA (Onpattro[®]) are approved by FDA for treatment of various diseases.

Applications of nanoparticles in drug delivery are also reported. Without being bound to theory, metal or non-metal nanoparticles may have such potential as long as their toxicity is not harmful. Examples of nanoparticles which may be applicable include, but are not limited to, gold, silver, silica, carbon, lipid nanoparticles and composite materials. In one embodiment, the nanoparticles are mesoporous such that the drug loading capacity may be increased. In one embodiment, the nanoparticles may be hollow. In one embodiment, the nanoparticles may have a core-shell structure.

The present disclosure also proposes that using short-chain PEG and optionally having a high density of PEG on the surface of the nano-sized carriers can avoid the attachment of anti-PEG antibodies and prevent or lessen negative effects as ABC phenomenon. The density-based design of the PEGlyated nano-size carriers can further improve immune tolerance, safety and timely use of PEGylated nanomedicine and nanovaccine. This approach is promising and may lead to a breakthrough in pharmaceutical applications as there are no available products in the market which contain nanoparticles functionalized with short-chain PEG. Instead, the commercially available nanocarrier, e.g., liposome in liposomal doxorubicin, uses long chains. This becomes a liability because anti-PEG antibodies can recognize the numerous ethylene oxide groups in long-chain PEG and bind to them. One gist of the inventive concepts lies in using short-chains PEGs to modify the nano-sized carriers.

However, PEG's shielding effect may also be dependent on the PEG conformation, which is determined by various factors such as the molecular weight (i.e., length) of PEG and graft density on the surface. It is feasible to establish a "brush-like" conformation of PEG because most of the surface area of the nanoparticle can be covered by the PEG in said confirmation. In contrast, a "mushroom-like" conformation of PEG will lead to partial exposure of the nanoparticle's surface, making the PEG easier to be detected by the immune system, e.g., anti-PEG antibody. Hence, it would be highly unfavorable to use short-chain PEGs for surface modification because the short-chain PEG will more easily form a mushroom-like conformation on the surface of the NPs, not the preferred brush-like conformation, which may have ability to avoid detection from the immune system, e.g., identification by anti-PEG antibodies.

Surprisingly, the inventors found that it is still possible to formulate nanoparticles with short-chain PEG to establish a "brush" structure, and doing so can avoid the problems brought by the long-chain PEG modification. This is because when there is a sufficient amount of PEG chains (i.e., enough high PEG density on the nanoparticle), the chains may push each other away, which will lead to an optimized conformation. Hence, short-chain PEGs will also have a chance to form the brush conformation as long as the surface PEG density is high enough. Despite this, it is also important to avoid "over-PEGylation," which would negatively impact the desired characteristics of nanoparticles, especially for soft nanoparticles, such as liposomes and polymers. In the case of liposomes, for example, it has been reported that the liposome structure will change from spherical to disc-like shape when the PEGylation level is too high. This change of shape may negatively affect the efficacy of drug delivery.

*In vitro* ELISA tests clarify the anti-PEG antibodies binding affinity with different length/density PEG functionalized MSNs. In one embodiment, the testing results may be considered indirect evidence for correlating the therapeutic efficacy and severe allergic reactions to the existence and degree of anti-PEG antibodies while PEGylated therapeutics are applied.

In another aspect, the MSNs noted in the embodiments mentioned above can be interchangeable with liposomes, micelles or other nano-sized carriers. That is, the disclosure relates to liposomes that were functionalized with varying types of PEG (e.g., MW less than 2,000) to yield short-chain PEG modified liposomes of various sizes and properties, etc.

The synthesis of nano-sized carriers, particularly MSNs, with physicochemical properties may be optimal. MSNs with different densities of PEG on the surface were synthesized for investigating the correlation between PEG density and anti-PEG antibody recognition. Various amounts (about 0.02 w/v to about 1.0 w/v, particularly, about 0.1 w/v to about 0.6 w/v) of PEG (MW less than 2,000) solution (preferably in ethanolic solution) is added to nano-sized carriers (such as MSNs) to form various PEG density. The resulting mixture is aged without stirring and then placed in an oven for more than 24 hours of hydrothermal treatment at 80°C to 95°C to form nano-carrier carrier with PEG density as described herein. One example of producing nano-sized carriers of the present disclosure is presented below. Initially, 0.44 g of CTAB was dissolved in 225 mL of ammonium hydroxide solution at the desired temperature (40-70°C for 25 to 50 nm) in a sealed beaker. After 15-minutes of stirring, the sealed membrane was removed, and then ethanolic TEOS were added to the solution under vigorous stirring (additionally added ethanolic RITC-conjugated APTMS for synthesis of RITC-conjugated MSN (RMSN)). After 30 to 60 minutes of stirring, the various amounts (> 82.5 uL) of PEG500-silane or 1.125 g, 0.563 g, and 0.375 g of PEG2000-silane in ethanolic solution was added for varying PEG density. After 1 hour, the mixture was aged at desired temperature 40-60°C without stirring for overnight. And then the solution was sealed and placed in an oven for 24 to 48 hours of hydrothermal treatment at 90 °C. The surfactant templates in the pores of the MSNs were extracted using ammonium nitrate. Then the nanoparticles were washed several times with ethanol, collected by cross-flow filtration and stored. For other functional group modified MSNs synthesis including MSN-PEG-TA (25 nm particle size, PEGylated with PEG500 and decorated with quaternary ammonium salts TA) and MSN-PEG500-PEI, etc. additionally added TA-silane, PEI-silane or other functional-silanes for surface modification. MSNs without PEGylation will also be synthesized to serve as a negative control. Particularly, PEGylated MSNs (25 nm particle size, PEGylated with PEG500 and decorated with quaternary ammonium salts TA) is illustrated in the disclosure.

The short-chain PEG functionalized MSNs can be loaded with a bioactive ingredient. In one embodiment, the bioactive ingredient is an anti-cancer agent or drug. Examples of the anti-cancer agent include, but are not limited to, everolimus, trabectedin, abraxane, TLK 286, AV-299, DN-I0I, pazopanib, GSK690693, RTA 744, ON 0910.Na, AZD 6244 (ARRY-142886),AMN-107, TKI-258, GSK461364, AZD 1152, enzastaurin, vandetanib, ARQ-197, MK-0457, MLN8054, PHA-739358, R-763, AT-9263, a FLT-3 inhibitor, a VEGFR inhibitor, an EGFR TK inhibitor, an aurora kinase inhibitor, a PIK-1 modulator, a Bcl-2 inhibitor, an HDAC inhibitor, a c-MET inhibitor, a PARP inhibitor, a Cdk inhibitor, an EGFR TK inhibitor, an IGFR-TK inhibitor, an anti-HGF antibody, a PI3 kinase inhibitors, an AKT inhibitor, a JAK/STAT inhibitor, a checkpoint-1 or 2 inhibitor, a focal adhesion kinase inhibitor, a Map kinase (mek) inhibitor, a VEGF trap antibody, pemetrexed, erlotinib, dasatinib, nilotinib, decatanib, panitumumab, amrubicin, oregovomab, Lep-etu, nolatrexed, azd2171, batabulin, ofatumumab, zanolimumab, edotecarin, tetrandrine, rubitecan, tesmilifene,oblimersen, ticilimumab, ipilimumab, gossypol, Bio 111, 131-I-TM-601, ALT-110, BIO 140, CC 8490, cilengitide, gimatecan, IL 13-PE38QQR, INO 1001, IPdR1 KRX-0402, lucanthone, LY 317615, neuradiab, vitespan, Rta 744, Sdx 102, talampanel, atrasentan, Xr 311, romidepsin, ADS-I00380, sunitinib,5-fluorouracil, vorinostat, etoposide, gemcitabine, doxorubicin, liposomal doxorubicin, 5'-deoxy-5-fluorouridine, vincristine, temozolomide, ZK-304709, seliciclib; PD0325901 , AZD-6244, capecitabine, L-Glutamic acid, N -[ 4-[2-(2-amino-4,7 -dihydro-4-oxo-1-H-pyrrolo[2,3-d]pyrimidin-5-yl) ethyl]benzoyl]- disodium salt, heptahydrate, camptothecin, PEG-labeled irinotecan, tamoxifen, toremifene citrate, anastrazole, exemestane, letrozole, DES(diethylstilbestrol), estradiol, estrogen, conjugated estrogen, bevacizumab, IMC-1C11, CHIR-258, 3-[5-(methylsulfonyl piperadinemethyl)-indolyl]-quinolone, vatalanib, AG-013736, AVE-0005, goserelin acetate, leuprolide acetate, triptorelin pamoate, medroxyprogesterone acetate, hydroxyprogesterone caproate, megestrol acetate, raloxifene, bicalutamide, flutamide, nilutamide, megestrol acetate, CP-724714; TAK-165, HKI-272, erlotinib, lapatanib canertinib, ABX-EGF antibody, erbitux, EKB-569, PKI-166, GW-572016, lonafarnib, BMS-214662, tipifamib; amifostine, NVP-LAQ824, suberoyl analide hydroxamic acid, valproic acid, trichostatin A, FK-228, SU11248, sorafenib, KRN951 , aminoglutethimide, amsacrine, anagrelide, L-asparaginase, Bacillus CalmetteGuerin (BCG) vaccine, bleomycin, buserelin, busulfan, carboplatin, carmustine, chlorambucil, cisplatin, cladribine, clodronate, cyproterone, cytarabine, dacarbazine, dactinomycin, daunorubicin, diethylstilbestrol, epirubicin, fludarabineetc. fludrocortisone, fluoxymesterone, flutamide, gemcitabine, hydroxyurea, idarubicin, ifosfamide, imatinib, leuprolide, levamisole, lomustine, mechlorethamine, melphalan, 6-mercaptopurine, mesna, methotrexate, mitomycin, mitotane, mitoxantrone, nilutamide, octreotide, oxaliplatin, pamidronate, pentostatin, plicamycin, porfimer, procarbazine, raltitrexed, rituximab, streptozocin, teniposide, testosterone,thalidomide, thioguanine, thiotepa, tretinoin, vindesine, 13-cis-retinoic acid, phenylalanine mustard, uracil mustard, estramustine, altretamine, floxuridine, 5-deooxyuridine, cytosine arabinoside, 6-mecaptopurine, deoxycoformycin, calcitriol, valrubicin, mithramycin, vinblastine, vinorelbine, topotecan, razoxin, marimastat, COL-3, neovastat, BMS-275291, squalamine, endostatin, SU5416, SU6668, EMD121974, interleukin-12, IM862, angiostatin, vitaxin, droloxifene, idoxyfene, spironolactone, finasteride, cimitidine, trastuzumab, denileukin diftitox, gefitinib, bortezimib, paclitaxel, cremophor-free paclitaxel, docetaxel, ixabepilone, epithilone B, BMS-247550, BMS-310705, droloxifene,4-hydroxytamoxifen, pipendoxifene,ERA-923, arzoxifene, fulvestrant, acolbifene, lasofoxifene, idoxifene, TSE-424, HMR-3339, ZK186619, topotecan, PTK787/ZK 222584, VX-745, PD 184352, rapamycin, 40-O-(2-hydroxyethyl)rapamycin, temsirolimus, AP-23573, RAD001, ABT-578, BC-210, LY294002, LY292223, LY292696, LY293684, LY293646, wortmarmin, ZM336372, L-779,450, PEG-filgrastim, darbepoetin, erythropoietin, granulocyte colony-stimulating factor, zolendronate, prednisone, cetuximab, granulocyte macrophage colony-stimulating factor, histrelin, pegylated interferon alfa-2a, interferon alfa-2a,pegylated interferon alfa-2b, interferon alfa-2b, azacitidine, PEG-L-Asparaginase, lenalidomide, gemtuzumab, hydrocortisone, interleukin-11, dexrazoxane, alemtuzumab, all-transretinoic acid, ketoconazole, interleukin-2, megestrol, immune globulin, nitrogen mustard, methylprednisolone, ibritgumomab tiuxetan, androgens, decitabine, hexamethylmelamine, bexarotene, tositumomab, arsenic trioxide, cortisone, editronate, mitotane, cyclosporine, liposomal daunorubicin, Edwina-asparaginase, strontium 89, casopitant, netupitant, an NK-1 receptor antagonists, palonosetron, aprepitant, diphenhydramine , hydroxyzine, metoclopramide, lorazepam, alprazolam, haloperidol, droperidol, dronabinol, dexamethasone, methylprednisolone, prochlorperazine, granisetron, ondansetron, dolasetron, tropisetron, pegfilgrastim, erythropoietin, epoetin alfa, curcumin, ALZ001, JM17 and darbepoetin alfa.

In one embodiment, the short-chain PEG functionalized MSNs may exhibit a blood circulation half-life of at least 2 hours, preferably at least 4 hours.

The present disclosure also relates to a drug delivery system, comprising a bioactive ingredient and the short-chain PEG functionalized nano-sized carrier.

The present disclosure also relates to a method of treating a disease in a subject in need, comprising administering the PEGylated nano-sized carrier loaded with non-PEGylated or PEGylated bioactive agent or the drug delivery system, as described herein, to a subject in need. In one embodiment, the disease is cancer. Examples of cancers include, but are not limited to, carcinomas (e.g., squamous-cell carcinomas, adenocarcinomas, hepatocellular carcinomas, and renal cell carcinomas), particularly those of the bladder, bone, bowel, breast, cervix, colon (colorectal), esophagus, head, kidney, liver (hepatocellular), lung, nasopharyngeal, neck, ovary, pancreas, prostate, and stomach; leukemias, such as acute myelogenous leukemia, acute lymphocytic leukemia, acute promyelocytic leukemia (APL), acute T-cell lymphoblastic leukemia, adult T-cell leukemia, basophilic leukemia, eosinophilic leukemia, granulocytic leukemia, hairy cell leukemia, leukopenic leukemia, lymphatic leukemia, lymphoblastic leukemia, lymphocytic leukemia, megakaryocytic leukemia, micromyeloblastic leukemia, monocytic leukemia, neutrophilic leukemia and stem cell leukemia; benign and malignant lymphomas, particularly Burkitt's lymphoma, Non-Hodgkin's lymphoma and B-cell lymphoma; benign and malignant melanomas; myeloproliferative diseases; sarcomas, particularly Ewing's sarcoma, hemangiosarcoma, Kaposi's sarcoma, liposarcoma, myosarcomas, peripheral neuroepithelioma, and synovial sarcoma; tumors of the central nervous system (e.g., gliomas, astrocytomas, oligodendrogliomas, ependymornas, glioblastomas, neuroblastomas, ganglioneuromas, gangliogliomas, medulloblastomas, pineal cell tumors, meningiomas, meningeal sarcomas, neurofibromas, and Schwannomas); germ-line tumors (e.g., bowel cancer, breast cancer, prostate cancer, cervical cancer, uterine cancer, lung cancer (e.g., small cell lung cancer, mixed small cell and non-small cell cancer, pleural mesothelioma, including metastatic pleural mesothelioma small cell lung cancer and non-small cell lung cancer), ovarian cancer, testicular cancer, thyroid cancer, astrocytoma, esophageal cancer, pancreatic cancer, stomach cancer, liver cancer, colon cancer, and melanoma; mixed types of neoplasias, particularly carcinosarcoma and Hodgkin's disease; and tumors of mixed origin, such as Wilms' tumor and teratocarcinomas, among others. Examples of cancers include, but are not limited to, carcinomas (e.g., squamous-cell carcinomas, adenocarcinomas, hepatocellular carcinomas, and renal cell carcinomas), particularly those of the bladder, bone, bowel, breast, cervix, colon (colorectal), esophagus, head, kidney, liver (hepatocellular), lung, nasopharyngeal, neck, ovary, pancreas, prostate, and stomach; leukemias, such as acute myelogenous leukemia, acute lymphocytic leukemia, acute promyelocytic leukemia (APL), acute T-cell lymphoblastic leukemia, adult T-cell leukemia, basophilic leukemia, eosinophilic leukemia, granulocytic leukemia, hairy cell leukemia, leukopenic leukemia, lymphatic leukemia, lymphoblastic leukemia, lymphocytic leukemia, megakaryocytic leukemia, micromyeloblastic leukemia, monocytic leukemia, neutrophilic leukemia and stem cell leukemia; benign and malignant lymphomas, particularly Burkitt's lymphoma, Non-Hodgkin's lymphoma and B-cell lymphoma; benign and malignant melanomas; myeloproliferative diseases; sarcomas, particularly Ewing's sarcoma, hemangiosarcoma, Kaposi's sarcoma, liposarcoma, myosarcomas, peripheral neuroepithelioma, and synovial sarcoma; tumors of the central nervous system (e.g., gliomas, astrocytomas, oligodendrogliomas, ependymornas, glioblastomas, neuroblastomas, ganglioneuromas, gangliogliomas, medulloblastomas, pineal cell tumors, meningiomas, meningeal sarcomas, neurofibromas, and Schwannomas); germ-line tumors (e.g., bowel cancer, breast cancer, prostate cancer, cervical cancer, uterine cancer, lung cancer (e.g., small cell lung cancer, mixed small cell and non-small cell cancer, pleural mesothelioma, including metastatic pleural mesothelioma small cell lung cancer and non-small cell lung cancer), ovarian cancer, testicular cancer, thyroid cancer, astrocytoma, esophageal cancer, pancreatic cancer, stomach cancer, liver cancer, colon cancer, and melanoma; mixed types of neoplasias, particularly carcinosarcoma and Hodgkin's disease; and tumors of mixed origin, such as Wilms' tumor and teratocarcinomas, among others.

The following examples are provided to make the present disclosure more comprehensible to those of ordinary skill in the art to which the present invention pertains, but are not intended to limit the scope of the invention.

## EXAMPLES

### Example 1

Here, liposome, mesoporous silica nanoparticle (MSN) and gold nanoparticle (AuNP) are chosen as model nano-sized carriers, each representing the organic, inorganic-non-metal and inorganic-metal nano-sized carrier.

In order to evaluate the function and action of PEG modification, PEG500 or PEG550 (with a weight average molecular weight of 500 g/mol or 550 g/mol) and PEG2000 (with a weight average molecular weight of 2,000 g/mol) are adopted.

*Liposome-PEG550 and Liposome-PEG2000*

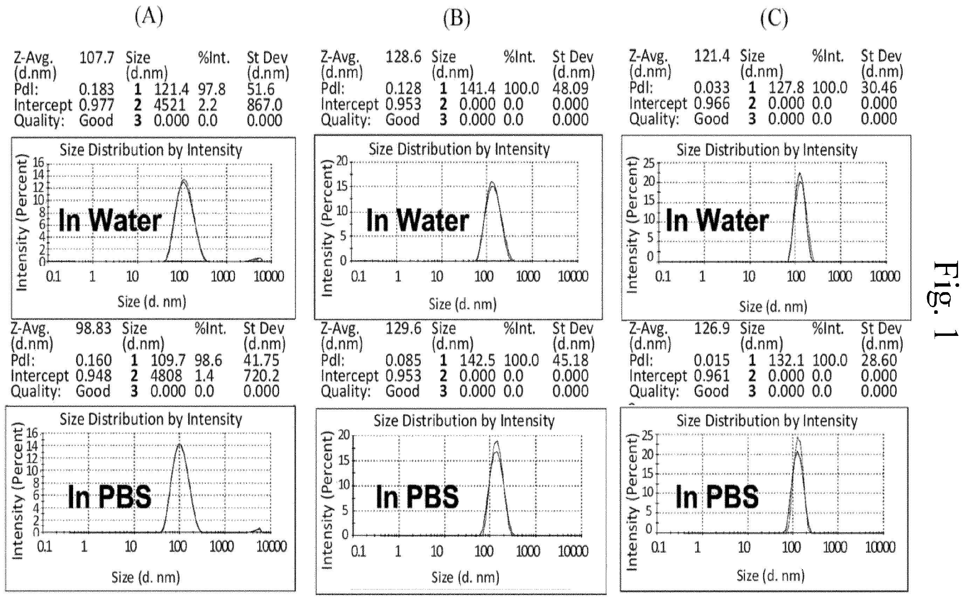

Liposome synthesized by the inventors was modified with PEG550 (Liposome-PEG550) or PEG2000 (Liposome-PEG2000). Commercial liposomal doxorubicin (Doxil, DOX-loaded Liposome with PEG2000 modification) was also purchased as a standard to evaluate the stability and characteristics of the self-synthesized liposome. Initially, HSPC, DSPE-PEG (mole% = 5.3% and PEG molecular weight is 2000 or 500), and cholesterol were dissolved in chloroform in an evaporating flask. Then the mixture solution was dried by the rotary evaporator. The dried lipid cake lay on the inside surface of a glass rotary flask. We reconstituted lipid solution with appropriate solution based on experimental requirement, rotating flask under 60 °C water bath for 1 hour. Next, the as-synthesized lipid underwent nano-size formation. The process was to freeze and thaw 10 times between liquid nitrogen and 70 °C water bath, staying in such conditions for 2 minutes each time. Finally, lipid solution was extruded through 200 nm filter membrane and 100 nm membrane 21 times respectively by mini extruder at 70 °C. Figure 1 shows DLS size distribution of Doxil (Fig. 1A), Liposome-PEG2000 (Fig. 1B) and Liposome PEG550 (Fig. 1C) in either water or PBS solution. They all exhibit a Z-average diameter of 100 nm to 120 nm, which means that the self-synthesized PEGylated liposome may be utilized in further applications.

*Mesoporous Silica Nanoparticle (MSN)-PEG500 and MSN-PEG2000*

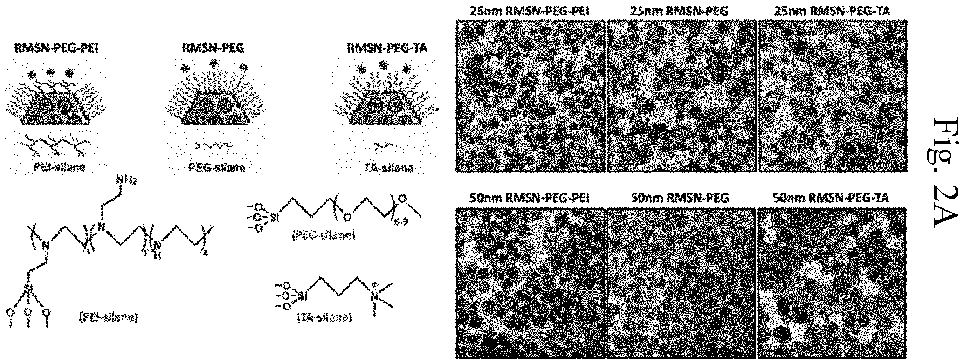

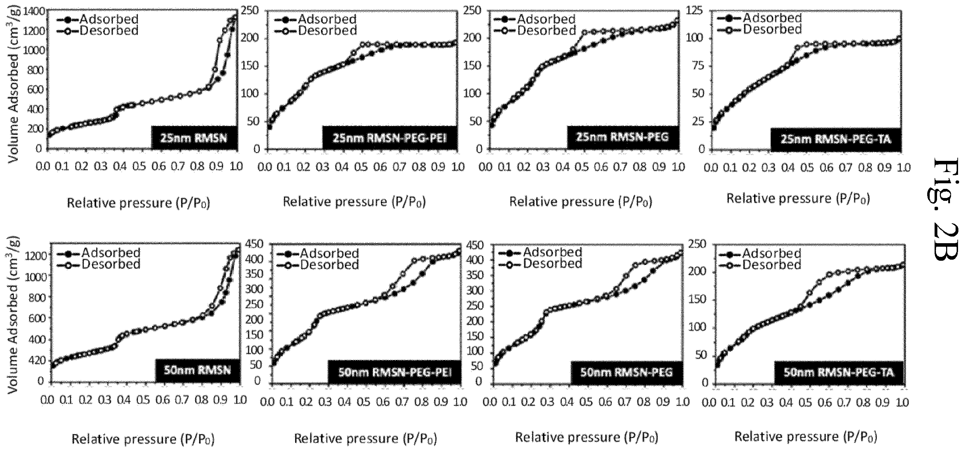

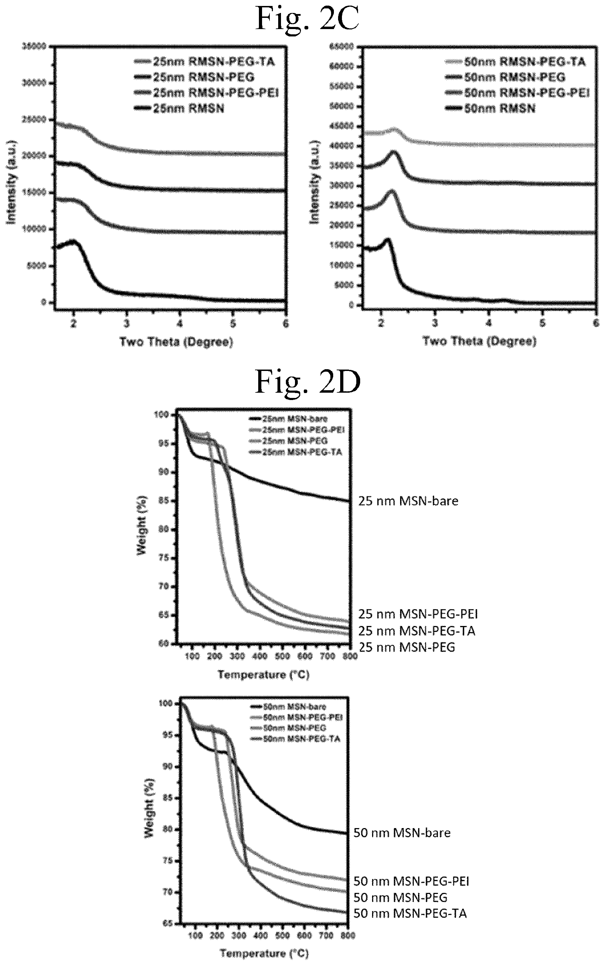

Mesoporous silica nanoparticles (MSNs) modified with PEG500 or PEG2000 were also synthesized. 0.44 g of CTAB was dissolved in 225 mL of ammonium hydroxide solutionat the desired temperature (40-70°C for 25 to 50 nm) in a sealed beaker. After 15-minutes of stirring, the sealed membrane was removed, and then ethanolic TEOS was added to the solution under vigorous stirring (additionally added ethanolic RITC-conjugated APTMS for synthesis of RITC-conjugated MSN (RMSN)). After 30 to 60 minutes of stirring, the various amounts (> 82.5 uL) of PEG500-silane or 1.125 g, 0.563 g, and 0.375 g of PEG2000-silane in ethanolic solution was added for varying PEG density. After 1 hour, the mixture was aged at desired temperature 40-60°C without stirring for overnight. And then the solution was sealed and placed in an oven for 24 to 48 hours of hydrothermal treatment at 90 °C. The surfactant templates in the pores of the MSNs were extracted using ammonium nitrate. Then the nanoparticles were washed several times with ethanol, collected by cross-flow filtration and stored. For other functional group modified MSNs synthesis including MSN-PEG-TA (25 nm particle size, PEGylated with PEG500 and decorated with quaternary ammonium salts TA) and MSN-PEG500-PEI, etc. additionally added TA-silane, PEI-silane or other functional-silanes for surface modification. Figure 2 shows characterizations of 25 nm and 50 nm MSNs-PEG500, MSNs-PEG(500)-TA, and MSNs-PEG(500)-PEI. The DLS size of 25 nm MSN-PEG500 and MSN-PEG2000 with different PEG densities are shown in Table 1 and Table 2. The nanoparticle would aggregate in the PBS condition if the particle synthesized with a decreased amount (density) of PEG. The DLS size of aggregated MSN-PEG500 particles synthesized with the decreased PEG amount (1/4X, 1/6X, 1/10X PEG) is larger than 200 nm in PBS. The DLS size of aggregated MSN-PEG2000 particles synthesized with the decreased PEG amount (1/3X PEG) is larger than 200 nm in PBS.

**Table 1**

| MSN-PEG500 | TEM (nm) | $D_h$ in PBS Z-average (d.nm) / PDI |
|---|---|---|
| MSN-bare | -- | -- |
| 1x PEG | 26.0 ± 4.5 | 38.7 / 0.090 |
| 1/2 PEG | 19.7 ± 2.8 | 70.8 / 0.244 |
| 1/3 PEG | 21.0 ± 3.6 | 104.3 / 0.219 |
| 1/4 PEG | 20.2 ± 3.0 | 707.5 / 0.346 |
| 1/6 PEG | 23.6 ± 4.0 | 1310/0.685 |
| 1/10 PEG | 23.3 ± 3.0 | 1721 / 0.709 |

**Table 2**

| MSN-PEG2000 | TEM (nm) | $D_h$ in PBS Z-average (d.nm) / PDI |
|---|---|---|
| 1x PEG | 22.1 ± 2.2 | 65.4 / 0.194 |
| 1/2 PEG | 21.0 ± 3.3 | 51.7 / 0.231 |
| 1/3 PEG | 21.3 ± 2.9 | 242.5 / 0.493 |

*Gold Nanoparticles (AuNP)- PEG500 and AuNP-PEG2000*

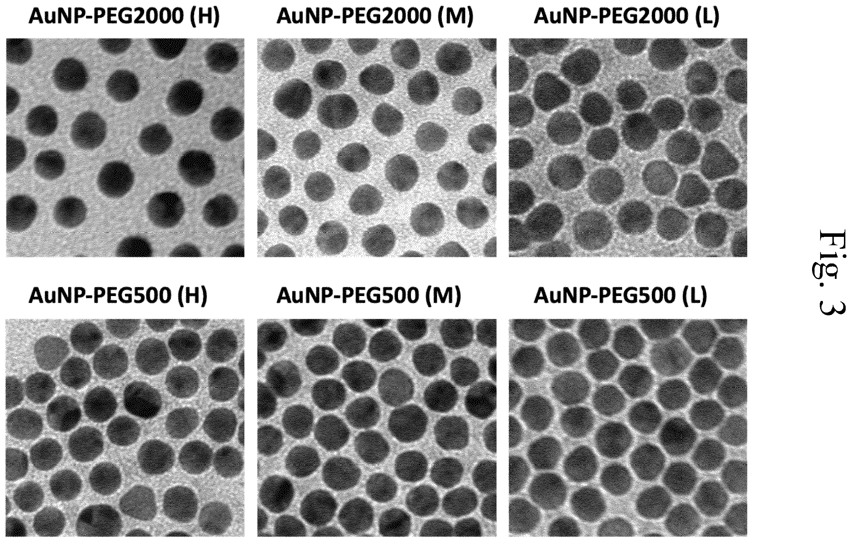

Gold nanoparticles were synthesized and modified with PEG500 or PEG2000, with different densities. Gold nanoparticles with 15 nm were synthesized by citrate reduction and seedling growth methods. 100 mL of 0.25 mM $HAuCl_4$ was heated to boil in a 250 mL Erlenmeyer flask. Next, 1 mL 3 % w/v sodium citrate was added under vigorous stirring. The color of the reaction solution changed from clear to purple, then finally became red. After that, the solution kept boiling and stirring for another 10 minutes, and then the solution was cooled on ice. The size of the as-synthesized gold nanoparticles was approximately 10-20 nm. After synthesis, the particles were coated with PEG. First, the gold nanoparticles were washed with buffer (750 µL of 0.25 mM sodium citrate with 0.05% v/v TWEEN20) 3 times before PEG grafting. The PEG density was modulated following published methods (J. Am. Chem. Soc. 2012, 134, 4, 2139), the gold nanoparticle solution was added PEG stock solution with 2000 Dalton molecular weight at concentrations of 0.83, 1.90, and 16.9 mM, that were used to give average PEG density of 0.48, 1.12, and 3.5 PEG/nm$^2$, respectively. After the mixture, the solution was incubated in a water bath at 60 °C for 1 hour. After that, the Au-PEG nanoparticles were washed twice with buffer to remove unbound PEG and collected by centrifugation. TEM images of the AuNP-PEG500 and AuNP-PEG2000 are shown in Figure 3.

Liposome-PEG550/PEG2000, MSN-PEG500 (1X PEG to 1/3X PEG)/PEG2000 (1X PEG to 1/2X PEG) and AuNP-PEG500/PEG2000 can exhibit stability without aggregation in PBS solution (the hydrodynamic size is smaller than 200 nm) that can be used in in vivo studies.

*Dynamic Light Scattering (DLS)*

Size measurements of the silica nanoparticles in different solution environments were performed with Dynamic Light Scattering (DLS) on a Malvern Zetasizer Nano ZS (Malvern, UK). The (solvated) particle sizes formed in different solutions were analyzed: $H_2O$, PBS buffer solution (pH7.4), and Dulbecco's Modified Eagle Medium (DMEM) with 10% FBS at room temperature.

*Transmission Electron Microscopy (TEM)*

Transmission electron microscopy (TEM) is used to directly examine and verify the appearance of the silica nanoparticles. The TEM images were taken on a Hitachi H-7100 transmission electron microscope operated at an accelerated 75-100 kV voltage. Samples dispersed in ethanol were dropped on carbon-coated copper grids and air-dried for TEM observation.

*Dox loaded MSN nanoparticle*

The Dox-loaded MSN nanoparticles were synthesized by incubating Dox with MSN solution for 1 hour, washing it twice with water to remove unloaded Dox and collecting by centrifugation or cross-flow system.

*N2 adsorption/desorption isotherms (BET / BJH)*

$N_2$ adsorption-desorption isotherms were determined using a Micrometric ASAP 2010 apparatus at 77 K under continuous adsorption conditions. Before the measurements, samples were degassed at $10^{-3}$ Torr and 110 °C for 16 h. The pore size distribution plots and pore volume were acquired from an analysis of the adsorption or desorption isotherms using the Barrett-Joyner-Halenda (BJH) method. The surface *area was acquired by a Brunauer-Emmett-Teller (BET) analysis.*

*Thermoaravimetric analysis (TGA):*

Thermogravimetric analysis (TGA) was recorded from 40 to 800 °C on a thermal analyzer with a heating rate of 10°C $min^{-1}$ in an air purge of 40 mL $min^{-1}$. Weight loss is determined in the range of 150-800 °C.

**Example 2**

*Density of PEG on nanoparticle*

Density of PEG on nanoparticles can be measured, evaluated and/or calculated in various manners. In one aspect, the surface PEG density on MSN was determined by quantifying the PEG content by weight using TGA and EA instruments relative to the total outer surface area determined by nitrogen sorption analysis and theoretical calculation. For example, the shape of MSN can be considered a cylinder, and the holes can be considered hollow channels. The surface area and the number of the channels can be calculated based on the size of MSN, pore size and porosity. Under this assumption, the surface area available for PEG modification can be obtained by summing the surface area of the cylinder and the surface area of the channels, thereby obtaining the surface PEG density. Here, the PEG density of MSN is about 0.5 to 5 PEG/$nm^2$. In one aspect, the synthesis of PEGylated gold nanoparticles was performed according to a previous report (J. Am. Chem. Soc. 2012, 134, 2139-2147), where the PEG grafting density was determined by multiplying the coordination efficiency by the grafting stoichiometry. Gold nanoparticles grafted with PEG at 3.5 (H), 1.12 (medium) and 0.48 (low) PEG/$nm^2$ were used here. In one aspect, the surface PEG density on liposomes was estimated based on the following published report (RSC Adv., 2018, 8, 7697). The theoretical determination of surface PEG conformation (mushroom or brush conformation) was determined based on the calculated ratio of the Flory dimension (Rf, equation 1) to the average distance between adjacent PEG chains (D, equation 2), where a is PEG monomer size in Å, N is degree of polymerization, A is the PEG area per lipid molecule in the bilayer and M is the mole fraction of PEG. The calculated Rf/D value of liposome-PEG2000 is about 1.06 and liposome-PEG500 is about 0.46, indicating the PEG conformation of liposome-PEG2000 and liposome-PEG500 in the mushroom regime. The correlation between molecular weight, grafting density, and the conformation of PEG chains (Nano Lett. 2021, 21, 1591) reveal the PEG density of the mushroom-brush intermediate conformation of PEG2000 would be 0.5 PEG/$nm^2$ and PEG density of mushroom conformation would be less than 0.5 PEG/$nm^2$.

$$\text{equation 1: } R_f = aN^{\frac{3}{5}}$$

$$\text{equation 2: } D = \left(\frac{A}{M}\right)^{\frac{1}{2}}$$

Taken together with the TGA and EA data, this calculation is further used to obtain the PEG density of different MSNs. The following tables represent the results.

Table 3

| Sample | TEM (nm) | $D_h$ in PBS Z-average (d.nm) / Pdl | N%, EA | Weight loss (%), TGA* | PEG density (#/nm$^2$) |
|---|---|---|---|---|---|
| 25nm MSN-PEG$_{500}$-PEI | 25.6 ±3.8 | 37.5 / 0.06 | 0.362 | 32.89 ( 24.73 ) | 3.53 |
| 25nm MSN-PEG$_{500}$ | 26.0 ±4.5 | 38.7/ 0.09 | - | 36.05 ( 27.89 ) | 4.43 |
| 25nm MSN-PEG$_{500}$-TA | 25.8 ±4.3 | 39.5/0.13 | 0.267 | 34.41 ( 26.25 ) | 3.75 |
| 50nm MSN-PEG$_{500}$-PEI | 48.4 ± 5.1 | 62.5/ 0.08 | 0.272 | 25.16 ( 10.48 ) | 2.29 |
| 50nm MSN-PEG$_{500}$ | 45.5 ± 5.3 | 54.4 / 0.05 | | 27.19 ( 12.51 ) | 2.87 |
| 50nm MSN-PEG$_{500}$-TA | 49.2 ±5.6 | 54.3/ 0.05 | 0.305 | 30.29 ( 15.61 ) | 3.44 |

Table 4

| 25nm MSN-PEG500 | TEM (nm) | Dh in PBS Z-average (d.nm) / PDI | Weight loss (%), TGA* | PEG density (#/nm$^2$) |
|---|---|---|---|---|
| 1x PEG (25nm MSN-PEG$_{500}$) | 26.0 ± 4.5 | 38.7 / 0.090 | 36.05 (27.89) | 4.43 |
| 1/2 PEG | 19.7 ±2.8 | 70.8 / 0.244 | 24.41 (16.25) | 1.68 |
| 1/3 PEG | 21.0 ±3.6 | 104.3 / 0.219 | 22.43 (14.27) | 1.54 |
| 1/4 PEG | 20.2 ±3.0 | 707.5 / 0.346 | 21.97 (13.81) | 1.43 |
| 1/6 PEG | 23.6 ± 4.0 | 1310/0.685 | 15.88 (7.72) | 0.87 |
| 1/10 PEG | 23.3 ± 3.0 | 1721 / 0.709 | 13.13 (4.97) | 0.54 |

*Weight loss is determined in the range of 150-800°C. The numbers in red with a pair of parentheses indicate the value after subtracting the background level.

## Example 3

### *In vitro* experiments

*Determination of the specific binding affinity between PEG-coated nanoparticle and anti-PEG antibody by ELISA*

The affinity between PEG-coated nanoparticles (Doxil, Liposome-PEG550/PEG2000, AuNP-PEG2000, and MSN-PEG500) and anti-PEG antibody was detected by enzyme-linked immunosorbent assays (ELISA). 96-well plates were coated with 50μL of 5 μg/mL anti-PEG IgM (AGP3) or 10 μg/mL anti-PEG IgG (6.3) for 2 hours. Next, added 200 µL of 5% milk dissolved in PBS and incubated at 4 °C overnight to wash out the non-adsorbed antibody. After incubation, the wells were washed with PBS three times, and then 50 µL PEG coated-nanoparticle sample was added and incubated at room temperature for 1 hour. After that, the wells were washed by PBS three times, then 50 µL diluted first detection antibody was added for one-hour incubation, repeated PBS washing for three times. Next, 50 µL diluted second detection antibody was added for one-hour incubation, also repeated PBS washing three times. Finally, 150 µL ABTS substrate solution was added to each well and incubated for 60 minutes, and then absorbance was measured by spectrophotometer at 405 nm.

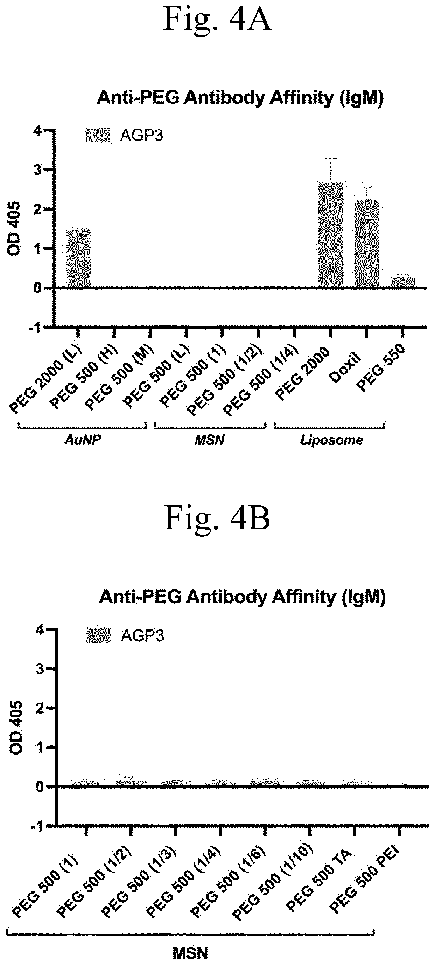

Next, AGP3 is considered capable of identifying the ethylene glycol segment in the PEG chain and thus can be used for verifying the PEG conformation on the surface of the nano-sized carriers. As shown in Figure 4A and Figure 4B, Doxil, Liposome-PEG2000, AuNP-PEG2000 showed high affinity toward AGP3. The affinity of liposome-PEG550 showed a significant decrease compared to liposome-PEG2000. In addition, AuNP-PEG500 (at three PEG density levels), MSN-PEG500 (at six PEG density levels), MSN-PEG500-TA, and MSN-PEG500-PEI showed nearly no affinity toward AGP3.

This reveals that both chain length of PEG and PEG density on the surface of nano-sized carriers would affect the affinity toward anti-PEG antibodies. In particular, nano-sized carriers modified with long-chain PEG (PEG2000), regardless of PEG density, would have an affinity toward anti-PEG antibodies. In addition, nano-sized carriers modified with short-chain PEG (PEG 500 or PEG550) at enough density would not have an affinity toward anti-PEG antibody; however, if the PEG density is too low (the density of PEG is less than 0.5 PEG/nm$^2$, e.g., Liposome-PEG in mushroom conformation), the PEG-modified nanocarriers would have a certain affinity toward anti-PEG antibody.

*Bridging the Gap between Mouse and Human Model* - *In vitro Human Serum Testing*

The inventors have already experimented with the binding affinity of anti-PEG IgM (AGP3) or anti-PEG IgG (6.3) to PEGylated MSNs *in vitro* by coating anti-PEG monoclonal antibodies on flat-bottom 96 well plates and then performing ELISA reader.

Their results show that the specially-formulated nanoparticles they have synthesized can avoid the recognition of mouse anti-PEG antibodies. These results encourage further study to see whether it is possible to have the same results in human models.

*Obtaining Blood from Healthy Donors and Checking for the Presence of Anti-PEG Antibodies*

Blood from ± 10 healthy donors was collected, and the plasma was procured. The plasma was then tested for anti-PEG antibodies through ELISA assay. Plasma that was confirmed to be seropositive was used for incubation with nanoparticles.

*Studying the Interaction between Different Nanoparticles and Anti-PEG Antibodies found in Seropositive Plasma*

PEGylated liposomal doxorubicin is a nanoformulation that is commonly used for treating cancer. Still, studies are showing that its efficacy can be reduced due to the aggregation with anti-PEG antibodies. Due to this phenomenon, they could be used as a positive control to test for the interaction with anti-PEG. PEGylated liposomal doxorubicin was incubated with seropositive human plasma and was consequently tested using ELISA assay for the extent of anti-PEG aggregation. The negative control for this experiment was the MSNs synthesized without PEGylation, for example, size 25 nm MSNs decorated with quaternary ammonium salts (henceforth called 25 nm MSN-TA).

Finally, 25 nm MSN-PEG500-TA and other MSNs of varying PEG length and density were incubated in seropositive plasma to let the anti-PEG bind. The ELISA assay was carried out to find the extent of binding. The results were compared to the controls.

**Example 4**

*In vivo* **Experiments**

*ELISA Assay for the Quantitative Analysis of the Generation of Anti-PEG Antibodies*

This test determines whether the repeated injections of different types of PEG-contained nanoparticles, biomolecules, etc., induce the production of anti-PEG antibodies. In this test, the injection of free PEG chains may also be used as a negative control as they are known as being non-immunogenic (hapten-like properties). Naïve mice (seronegative for anti-PEG antibodies) were injected with test articles a total of three times: day 0, day 7 and day 14. On the 17th day after the first injection, blood samples from each of the mice were collected, subsequently analyzed to quantify IgM or IgG anti-PEG antibodies using ELISA.

*In vivo Tumor-Inhibition Study in Mice that are Seropositive for the anti-PEG Antibody vs. Naïve Mice*

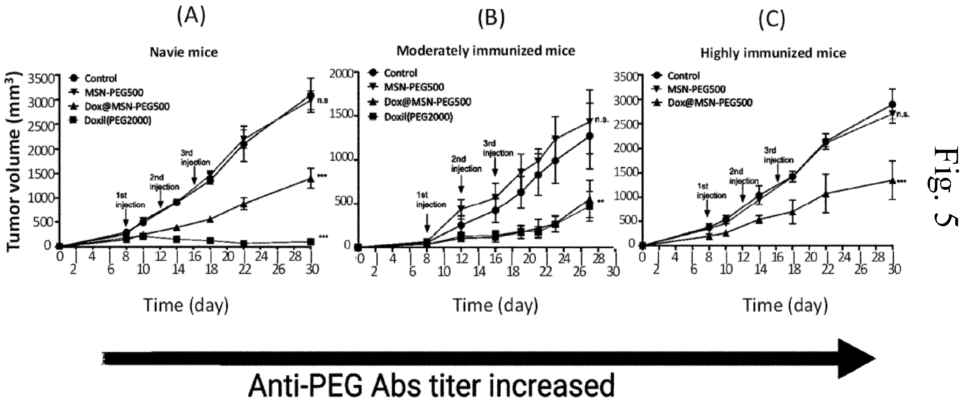

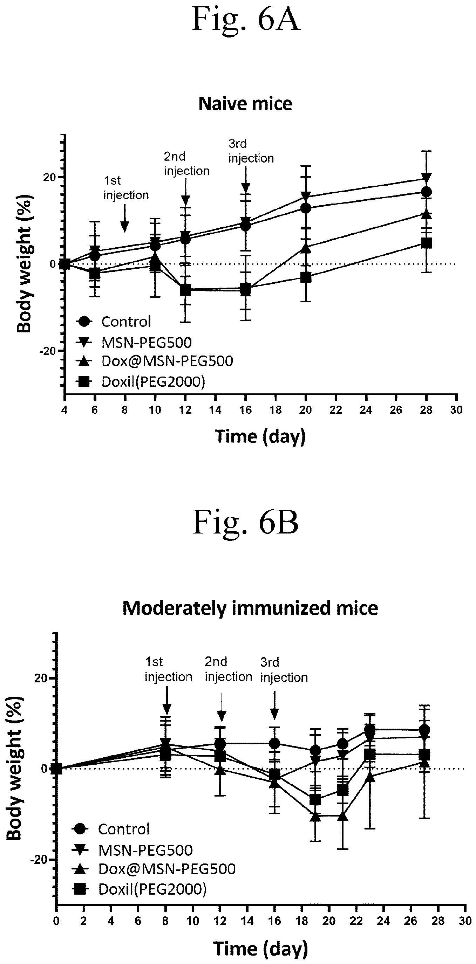

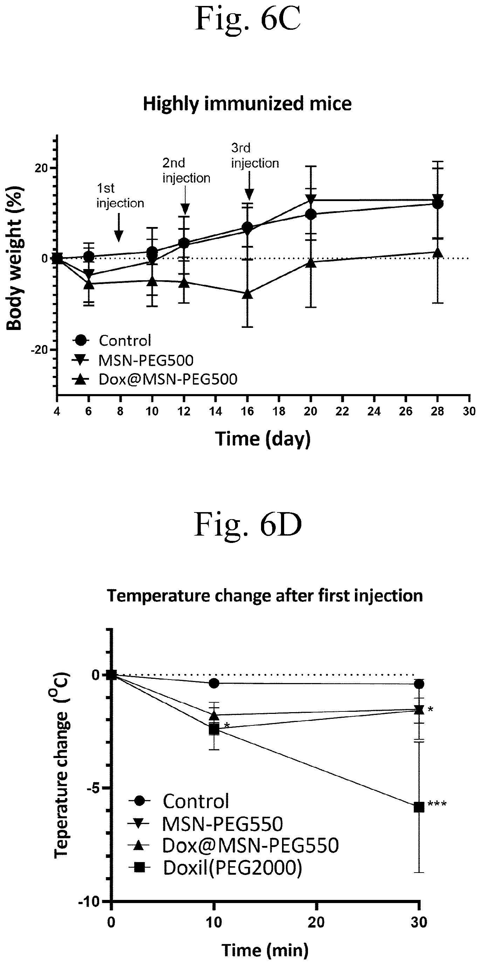

The inventors would like to determine if nanoparticles with specific physical properties can effectively inhibit the growth of tumors using a classical in vivo mouse xenograft model. To test the efficacy of nanoparticles, e.g., the ability not to induce the PEG immune response (causing side effects) and evading the ABC phenomenon (no efficacy reduction), the studies were performed on both naïve and seropositive mice based on the methods described below. Immunocompetent mice were immunized with BSA-PEG and OVA-PEG each for zero, one or two injections to induce naïve and anti-PEG antibody seropositive mice (moderate immune induction group: total 2 boost or high immune induction group: total 4 boosts). All mice were xenografted with 4T1 tumors and divided into three groups: naïve mice, moderately immunized mice (2 boosts) and highly immunized mice (4 boosts). All mice were injected with PBS, MSN-PEG500, Dox@MSN-PEG500, and Doxil (PEGylated liposomal doxorubicin) 3 times at 4-days intervals, and the injection started on day 8 after tumor implantation. Body weights and tumor volumes of the mice were measured twice a week. We also measured the anal temperature to evaluate body temperature at 10 minutes, 20 minutes, and 30 minutes after the first injection and the body temperature before injection was measured to be the baseline. According to the results, in naïve mice group, the mice treated with Dox@MSN-PEG500 or Doxil (liposomal doxorubicin with PEG2000 modification on the surface) exhibit inhibition of tumor growth (Doxil has better anti-cancer efficacy than Dox@MSN-PEG500) and bodyweight reduction during the therapeutic period but recovered after dosing was stopped. However, in the moderate immune induction mice (2 boosts) groups, the efficacy of Doxil was significantly reduced from better than Dox@MSN-PEG500 to as same as Dox@MSN-PEG500 (the tumor size of Doxil group is about 20 times smaller than the control group in naïve mice, but only 1/2 to 1/3 smaller than the control group in moderate immune induction mice), no additional adverse effects were observed. This result demonstrated that the pre-existing anti-PEG antibody in mice affects the efficacy of Doxil, but does not affect the efficacy of Dox@MSN-PEG500 (the tumor size of Dox@MSN-PEG500 group is about 1/2 to 1/3 smaller than the control group not only in naïve mice but also in moderate immune induction mice). The reduction of efficacy is caused by the ABC phenomenon that was previously mentioned (Figure 5A to 5C). In addition, the body temperature of Doxil treated mice sharply dropped 5 to 10 °C within 30 minutes after the first injection that is the symptom of (pseudo-)anaphylaxis, furthermore, the level of anaphylaxis is in a dose-dependent manner with the amount of anti-PEG antibody in the mice, severe (pseudo-)anaphylaxis was emerged in the high immune induction mice group, Doxil-treated mice all died within 30 mins after the first injection (Figure 6A to 6D). All data revealed that anti-PEG antibodies bind to the PEG2000 on the surface of Doxil to form an immune complex, which seems to activate the immune response to cause the allergic reaction and the complex would be recognized by immune cells and facilitated blood clearance leading to reduction of anti-cancer efficacy. In contrast, Dox@MSN-PEG500 did not induce significant anaphylaxis and body temperature change in moderate and high immune induction mice. Meanwhile, anti-cancer efficacy is consistent in naïve mice, moderate immune induction mice, and even high immune induction mice. In conclusion, the different molecular weights and densities of PEG on Doxil and Dox@MSN-PEG500 make opposite exhibitions in in vivo studies. The PEG2000 molecule with low density (mushroom-like conformation) on Doxil makes the anti-PEG antibody easily recognizes the particles and result in efficacy reduction and anaphylaxis induction. Oppositely the PEG500 molecule with high density on MSN-PEG500 isn't recognized by an anti-PEG antibody that means the MSN-PEG500 particle can evade ABC phenomenon and immune response induction, importantly remain the efficacy without anaphylaxis induction.

*Using Two-Photon Fluorescence (TPF) Spectroscopy to Investigate the Circulation Time of Different Nanoparticles in Blood*

The nanoparticles (PEGylated MSNs, PEGylated liposomal doxorubicin, and negative control) were first functionalized with rhodamine B isothiocyanate (RITC) as a fluorescent marker so that they can be detected by TPF spectroscopy. Afterward, the mice were given anesthetic (isoflurane 2.5%) and maintained in 1.5% isoflurane for the duration of the experiment. The RITC-functionalized nanoparticles were then injected intravenously into the ear, and the blood vessels of the ear shall be monitored. A selected area was monitored for up to 2 hours, which would then be followed by the taking of images using a FVMPE-RS multimode multiphoton scanning microscope (Olympus) at predetermined time intervals. The circulation time of all nanoparticles was compared.

*Using Non-Invasive In Vivo Imaging System (IVIS) to Study Biodistribution and Tumor- Targeting Efficiency of Different Nanoparticles*

The previously mentioned nanoparticles were injected into the tail vein of BALB/c mice bearing a 4T1 tumor. After a predetermined amount of time, the mice were sacrificed and their major organs and tumor shall be harvested.

The imaging was performed using the Xenogen IVIS-200 system.

Furthermore, the whole blood of the mice was collected in an anticoagulating EDTA-coated tube and subsequently analyzed using IDEXX ProCyte Dx® Hematology Analyzer. Mice serum aliquots were also analyzed using Fuji Dri-chem 4000i Analyzer for biochemical analysis of blood chemistry. This was done in order to assess the safety of the nanoparticle products when they are circulating in the blood.

**Testing the Capability of Nanoparticles to Evade ABC Phenomenon - Studying the Biological Effects upon Repeated Injections**

In order to test the long-term ability of the nanoparticles to resist the ABC phenomenon and to study the body's reaction to them, it is necessary to perform experiments over a significantly extended period. This will simulate the long-term dosing that chemotherapy patients undergo for their treatment. The various nanoparticles were administered twice to the mice in intervals of 3, 7 and 14 days. Additional injections may be performed if required. Afterward, several analytical techniques were used to assess the pharmacokinetics of the nanoparticles and the elicited biological effects.

*TPF Spectroscopy for Investigation of Blood Circulation Time of Nanoparticles*

This experiment can truly test the effect of the ABC phenomenon on the different types of nanoparticles that were previously mentioned. The nanoparticles were functionalized with RITC for TPF Spectroscopy imaging. They were then injected into seropositive mice (positive for anti-PEG antibodies). The blood circulation times of the first and second administration of RITC-nanoparticles were evaluated at 3, 7 and 14 days after the first injection.

*IVIS Imaging for Investigation of Nanoparticle Biodistribution and Tumor-Targeting Efficiency*

The most important parameter of anti-cancer medicine is the ability to be distributed into the tumor while avoiding biodistribution into other major organs. As such, this experiment can test the abilities of the different nanoparticles using IVIS imaging after the first and second administration at 3, 7 and 14 days post-first injection into seropositive mice.

A person of ordinary skill in the art of the subject invention should understand that variations and modifications may be made to the teaching and the disclosure without departing from the spirit and scope of the subject application. Based on the contents above, the subject application intends to cover any variations and modifications thereof with the proviso that the variations or modifications fall within the scope as defined in the appended claims or their equivalents.

**Claims**

1. A nano-sized carrier having surface modification with short-chain PEG for use of prevention of induction of producing an anti-PEG antibody or prevention of identification by an anti-PEG antibody, wherein the nano-sized carrier is loaded with a bioactive agent, wherein the short-chain PEG has less than 45, preferably about 2 to about 17, repeating units of oxyethylene ($O-CH_2-CH_2$), or wherein the short-chain PEG has a molecular weight less than 2,000 Da, preferably less than 1,000 Da or less than 750 Da.

2. The nano-sized carrier for use of claim 1, wherein the density of the short-chain PEG on the surface of the nano-sized carrier within the range from 0.5 to $7/nm^2$, preferably from 1 to $5/nm^2$.

3. The nano-sized carrier for use of any preceding claims, wherein the PEGylated nano-sized carrier has the short-chain PEG having an apparent size ranging from about 10 nm to about 200 nm, preferably 25 nm to 150 nm.

4. The nano-sized carrier for use of any preceding claims, wherein the prevention of inducing production of an anti-PEG antibody or identification by an anti-PEG antibody can reduce or eliminate binding of a PEGylated nano-sized carrier to an anti-PEG antibody and/or prevent reduction of effect or efficacy of a PEGylated bioactive agent or carrier or reduce adverse events caused by the anti-PEG antibody.

5. The nano-sized carrier for use of any preceding claims, wherein the PEGylated nano-sized carrier can be organic, inorganic, polymeric or metallic nanostructure, preferably wherein the PEGylated nano-sized carrier is solid or hollow.

6. The nano-sized carrier for use of any preceding claims, wherein the PEGylated nano-sized carrier is a silica nanoparticle, liposome or a metal (oxide) nanoparticle.

7. The nano-sized carrier for use of any preceding claims, wherein the nanoparticle is mesoporous.

8. The nano-sized carrier for use of any preceding claims, wherein the nano-sized carrier has a DLS particle size ranging from 10 nm to 200 nm, preferably 25 nm to 150 nm, in aqueous solutions, PBS or physiological condition.

9. The nano-sized carrier for use of any preceding claims, wherein the bioactive agent is a PEGylated bioactive agent or a non-PEGylated bioactive agent.

10. The nano-sized carrier for use of any preceding claims, wherein two or more bioactive agents are loaded to the nano-sized carrier.

11. The nano-sized carrier for use of any preceding claims, wherein the bioactive agent is a small molecule drug, a large molecule drug, a vaccine, an enzyme or an immunogen.

12. A nano-sized carrier having surface modification with short-chain PEG, wherein the short-chain PEG has less than 17 repeating units of oxyethylene (O-$CH_2$-$CH_2$) and has a density on the surface of the nano-sized carrier in the range from 0.5 to 7/nm$^2$, preferbaly from 1 to 5/nm$^2$.

13. The nano-sized carrier of claim 12, wherein the short-chain PEG described herein has a molecular weight less than 1000 Da, preferably less than 750 Da.

14. The nano-sized carrier of claims 12-13, wherein the PEGylated nano-sized carrier is a silica nanoparticle, liposome or a metal (oxide) nanoparticle, preferably being solid or hollow.

15. A drug delivery system, comprising the nano-sized carrier of anye of claims 12-14 and a bioactive agent.

(A)

Z-Avg. 107.7 Size %Int. St Dev
(d.nm) (d.nm) (d.nm)
PdI: 0.183 1 121.4 97.8 51.6
Intercept 0.977 2 4521 2.2 867.0
Quality: Good 3 0.000 0.0 0.000

Size Distribution by Intensity

In Water

Intensity (Percent) vs Size (d. nm)

Z-Avg. 98.83 Size %Int. St Dev
(d.nm) (d.nm) (d.nm)
PdI: 0.160 1 109.7 98.6 41.75
Intercept 0.948 2 4808 1.4 720.2
Quality: Good 3 0.000 0.0 0.000

Size Distribution by Intensity

In PBS

Intensity (Percent) vs Size (d. nm)

(B)

Z-Avg. 128.6 Size %Int. St Dev
(d.nm) (d.nm) (d.nm)
PdI: 0.128 1 141.4 100.0 48.09
Intercept 0.953 2 0.000 0.0 0.000
Quality: Good 3 0.000 0.0 0.000

Size Distribution by Intensity

In Water

Intensity (Percent) vs Size (d. nm)

Z-Avg. 129.6 Size %Int. St Dev
(d.nm) (d.nm) (d.nm)
PdI: 0.085 1 142.5 100.0 45.18
Intercept 0.953 2 0.000 0.0 0.000
Quality: Good 3 0.000 0.0 0.000

Size Distribution by Intensity

In PBS

Intensity (Percent) vs Size (d. nm)

(C)

Z-Avg. 121.4 Size %Int. St Dev
(d.nm) (d.nm) (d.nm)
PdI: 0.033 1 127.8 100.0 30.46
Intercept 0.966 2 0.000 0.0 0.000
Quality: Good 3 0.000 0.0 0.000

Size Distribution by Intensity

In Water

Intensity (Percent) vs Size (d. nm)

Z-Avg. 126.9 Size %Int. St Dev
(d.nm) (d.nm) (d.nm)
PdI: 0.015 1 132.1 100.0 28.60
Intercept 0.961 2 0.000 0.0 0.000
Quality: Good 3 0.000 0.0 0.000

Size Distribution by Intensity

In PBS

Intensity (Percent) vs Size (d. nm)

Fig. 1

# Fig. 2A

# Fig. 2B

## Fig. 2C

## Fig. 2D

# Fig. 3

# Fig. 4A

**Anti-PEG Antibody Affinity (IgM)**

# Fig. 4B

**Anti-PEG Antibody Affinity (IgM)**

# Fig. 6A

**Naive mice**

# Fig. 6B

**Moderately immunized mice**

# Fig. 6C

**Highly immunized mice**

# Fig. 6D

**Temperature change after first injection**

Europäisches
Patentamt

European
Patent Office

Office européen
des brevets

**EUROPEAN SEARCH REPORT**

Application Number

EP 21 21 8381

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 741 362 A2 (NANO TARGETING & THERAPY BIOPHARMA INC [TW]) 25 November 2020 (2020-11-25) * paragraph [0005] – paragraph [0008] * * examples * * claims * | 1-15 | INV. A61K9/127 A61K9/51 |
| X | HAYNES MATTHEW T. ET AL: "Maximizing the Supported Bilayer Phenomenon: Liposomes Comprised Exclusively of PEGylated Phospholipids for Enhanced Systemic and Lymphatic Delivery", APPLIED MATERIALS & INTERFACES, vol. 8, no. 37, 9 September 2016 (2016-09-09), pages 24361-24367, XP055920620, US ISSN: 1944-8244, DOI: 10.1021/acsami.6b05534 * abstract * * paragraph [02.2] – paragraph [02.4] * * paragraph [03.1] – paragraph [03.2]; figure 2; table 1 * * paragraph [0004] * | 1-15 | |

-/--

| | |
|---|---|
| | TECHNICAL FIELDS SEARCHED (IPC) |
| | A61K |

The present search report has been drawn up for all claims

| Place of search | Date of completion of the search | Examiner |
|---|---|---|
| The Hague | 17 May 2022 | Epskamp, Stefan |

EPO FORM 1503 03.82 (P04C01)

Europäisches
Patentamt

European
Patent Office

Office européen
des brevets

# EUROPEAN SEARCH REPORT

Application Number

EP 21 21 8381

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FONSECA LEANDRO C. ET AL: "How does the chain length of PEG functionalized at the outer surface of mesoporous silica nanoparticles alter the uptake of molecules?", NEW JOURNAL OF CHEMISTRY, vol. 40, no. 9, 1 January 2016 (2016-01-01), pages 8060-8067, XP055920643, GB ISSN: 1144-0546, DOI: 10.1039/C6NJ01316C Retrieved from the Internet: URL:https://pubs.rsc.org/en/content/articlepdf/2016/nj/c6nj01316c> * abstract * * page 8061, left-hand column, last paragraph – right-hand column, paragraph 3 * * table 1 * | 1-15 | |
| X | YASUYUKI SADZUKA ET AL: "Effects of mixed polyethyleneglycol modification on fixed aqueous layer thickness and antitumor activity of doxorubicin containing liposome", INTERNATIONAL JOURNAL OF PHARMACEUTICS, vol. 238, no. 1-2, 1 May 2002 (2002-05-01), pages 171-180, XP055053916, ISSN: 0378-5173, DOI: 10.1016/S0378-5173(02)00075-3 * abstract * * paragraph [02.2]; table 2 * | 1-15 | TECHNICAL FIELDS SEARCHED (IPC) |

-/--

The present search report has been drawn up for all claims

| Place of search | Date of completion of the search | Examiner |
|---|---|---|
| The Hague | 17 May 2022 | Epskamp, Stefan |

EPO FORM 1503 03.82 (P04C01)

Europäisches
Patentamt

European
Patent Office

Office européen
des brevets

# EUROPEAN SEARCH REPORT

Application Number

EP 21 21 8381

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DOS SANTOS ET AL: "Influence of poly(ethylene glycol) grafting density and polymer length on liposomes: Relating plasma circulation lifetimes to protein binding", BIOCHIMICA ET BIOPHYSICA ACTA, ELSEVIER, AMSTERDAM, NL, vol. 1768, no. 6, 24 May 2007 (2007-05-24), pages 1367-1377, XP022095186, ISSN: 0005-2736, DOI: 10.1016/J.BBAMEM.2006.12.013 * abstract * * paragraph [02.1] - paragraph [02.2] * * paragraph [03.1]; figure 2; table 1 * * page 1374, left-hand column, last paragraph - right-hand column, last paragraph; figure 7 * | 1-15 | |
| X | SIMPSON CARRIE A. ET AL: "Short-Chain PEG Mixed Monolayer Protected Gold Clusters Increase Clearance and Red Blood Cell Counts", ACS NANO, vol. 5, no. 5, 19 April 2011 (2011-04-19), pages 3577-3584, XP055920634, US ISSN: 1936-0851, DOI: 10.1021/nn103148x * abstract * * page 3582, left-hand column, paragraph 2 - paragraph 3 * | 1-15 | TECHNICAL FIELDS SEARCHED (IPC) |

-/--

The present search report has been drawn up for all claims

| Place of search | Date of completion of the search | Examiner |
|---|---|---|
| The Hague | 17 May 2022 | Epskamp, Stefan |

EPO FORM 1503 03.82 (P04C01)

# EP 4 023 211 A1

Europäisches
Patentamt
European
Patent Office
Office européen
des brevets

## EUROPEAN SEARCH REPORT

**Application Number**

EP 21 21 8381

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | QI YANG ET AL: "Evading Immune Cell Uptake and Clearance Requires PEG Grafting at Densities Substantially Exceeding the Minimum for Brush Conformation", MOLECULAR PHARMACEUTICS, vol. 11, no. 4, 25 March 2014 (2014-03-25), pages 1250-1258, XP055675707, US ISSN: 1543-8384, DOI: 10.1021/mp400703d * abstract * * page 1251, left-hand column, paragraph 2 * * paragraph [02.1] * * paragraph [03.1] – paragraph [03.2]; figures 1-3 * * paragraph [0004] – paragraph [0005] * | 1-15 | |
| A | JUNG SOO SUK ET AL: "PEGylation as a strategy for improving nanoparticle-based drug and gene delivery", ADVANCED DRUG DELIVERY REVIEWS, vol. 99, 1 April 2016 (2016-04-01), pages 28-51, XP055551449, Amsterdam , NL ISSN: 0169-409X, DOI: 10.1016/j.addr.2015.09.012 * abstract * * paragraphs [2.3.1], [2.3.2] * * paragraph [02.5] * * paragraph [04.3] * | 1-15 | TECHNICAL FIELDS SEARCHED (IPC) |

The present search report has been drawn up for all claims

| Place of search | Date of completion of the search | Examiner |
|---|---|---|
| The Hague | 17 May 2022 | Epskamp, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 21 8381

17-05-2022

| Patent document cited in search report | | Publication date | Patent family member(s) | | Publication date |
|---|---|---|---|---|---|
| EP 3741362 | A2 | 25-11-2020 | CA | 3080024 A1 | 03-11-2020 |
| | | | CN | 111870699 A | 03-11-2020 |
| | | | EP | 3741362 A2 | 25-11-2020 |
| | | | JP | 2020200306 A | 17-12-2020 |
| | | | TW | 202106339 A | 16-02-2021 |
| | | | US | 2020345649 A1 | 05-11-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Analytical Chemistry,* 2016, vol. 88 (21), 10661-10666
- *ACS Nano,* 2021, vol. 15 (9), 14022-14048
- *J. Am. Chem. Soc.,* 2012, vol. 134 (4), 2139
- *J. Am. Chem. Soc.,* 2012, vol. 134, 2139-2147
- *RSC Adv.,* 2018, vol. 8, 7697
- *Nano Lett.,* 2021, vol. 21, 1591